# EUROPEAN PATENT APPLICATION

(11) **EP 1 743 663 A2**
(43) Date of publication of application: **17.01.2007**
(21) Application number: 06253374.0
(22) Date of filing: 28.06.2006
(51) Int. Cl.: A61L 27/38, A61L 27/54

(54) **Multi-compartment delivery system**

(30) Priority: 29.06.2005 US 170410
(71) Applicant: LifeScan, Inc., Milpitas, CA 95035 (US)
(72) Inventor: Ghabrial, Ragae, Helmetta, NJ 08828 (US); Fung, Ramie, Flemington, NJ 08822 (US); Rezania, Alireza, Hillsborough NJ 08844 (US); Davis, Janet, Branchburg, NJ 08876 (US)
(74) Representative: Tunstall, Christopher Stephen

(57) **Abstract**

This invention provides devices designed to effectively deliver multiple biological entities in combination for tissue engineering. In particular, the present invention provides devices capable of delivering cells or clusters of cells, such as islets of Langerhans, in combination with a therapeutic compound, such as an angiogenic growth factor, for the purpose of transplantation. The devices of the present invention are composed of at least two compartments that are designed independently and processed separately in order to accommodate different requirements of the biological entities. The compartments of the present device can be combined prior to or at the time of implantation, such that the therapeutic released from one compartment provides some benefit to cells hosted by another compartment to promote or improve their proliferation, differentiation, survival, or functionality.

## Description

### FIELD OF THE INVENTION

This invention relates generally to biocompatible devices for delivery of drugs and cells, and in particular to implantable biocompatible matrices suitable for delivery of therapeutic compounds in combination with organs, tissues, or cells.

### BACKGROUND OF THE INVENTION

Tissue engineering strategies have explored the use of biomaterials in combination with cells and/or growth factors to develop biological substitutes that ultimately can restore or improve tissue function. Scaffold materials have been extensively studied as tissue templates, conduits, barriers and reservoirs useful for tissue repair. In particular, synthetic and natural materials in the form of foams, sponges, gels, hydrogels, textiles and nonwovens have been used *in vitro* and *in vivo* to reconstruct or regenerate biological tissue, as well as deliver chemotactic agents for inducing tissue growth. See, for example, U.S. Patent Nos. 5,770,417, 6,022,743, 5,567,612, 5,759,830, 6,626,950, 6,534,084, 6,306,424, 6,365,149, 6,599,323, 6,656,488, and 6,333,029.

It is desirable for a scaffold to possess some fundamental characteristics such as being biocompatible, having adequate mechanical strength to resist loads experienced during surgery; being pliable, being highly porous to allow cell invasion or growth, being able to allow increased retention of cells in the scaffold, being easily sterilized, being susceptible to remodeling by invading tissue, and being degradable as the new tissue is formed. It is also desirable for a scaffold to have the ability to deliver certain therapeutics with specific activities and desirable release kinetics, for example, to reduce inflammation at the transplant site, to down regulate invading immune cells, to enhance proliferation of transplanted cells, or to induce differentiation of transplanted cells into functional tissue. In other cases, a scaffold may carry more than one biological entity such as cells, cell clusters, or organoids, in which case the scaffold must possess variable characteristics to accommodate each entity. It is clear that the manufacture of scaffold as a replacement for diseased or damaged tissue requires flexible designs to accommodate the complexity and high specificity associated with various biological functions.

Tissue engineering may offer alternative, promising approaches for treating diabetes. Degradable or non-degradable matrices have been used to seed and culture islet cells for implantation *in vivo.* As islet survival depends on diffusion of oxygen and nutrients, establishing stable vascularization is critical to islet survival. It has been shown that a sustained release of angiogenic signals is required for establishing stable vascularization, which allows for the development of a permanent implant structure. Efforts have been made to encapsulate, with a non-degradable material, both islet cells and angiogenic factors suspended in a matrix material such as gelatin hydrogel. Scaffold constructs made of biodegradable materials have also been used for delivering islet cells into various anatomical sites *in vivo.* For example, nonwoven scaffolds are an ideal matrix for hosting islets, as they provide adequate support for islets to get entangled within the fibers and allow enough porosity for diffusion of oxygen and nutrients, which is essential especially for the preliminary stages of the transplantation process until the vasculature is established. On the other hand, incorporation of a therapeutic compound into a nonwoven scaffold is fairly inefficient, since nearly 90% of the nonwoven scaffold can be void air. To address this issue, composite scaffolds have been designed to incorporate a foam component which lowers the overall porosity of the scaffold, yet may compromise the ability of the scaffold to host islets.

Clearly, there is a need for scaffold constructs that permit efficient delivery of multiple, distinct biological entities, such as islet cells and a therapeutic compound.

### SUMMARY OF THE INVENTION

The present invention is directed to a biocompatible, implantable, partially or fully biodegradable delivery device, which is composed of at least two compartments. The compartments are designed independently and processed separately to accommodate and effectively deliver two or more biologically relevant entities.

In one embodiment, at least two compartments of a device are loaded separately with a therapeutic compound and cells, respectively, such that the therapeutic compound released from one compartment provides a beneficial activity to the cells hosted by another compartment.

In another embodiment, at least two compartments of a device are loaded separately with two types of cells, wherein the cells in one compartment provide a beneficial effect on the cells hosted by another compartment. For example, one compartment of a device can be loaded with cells that protect cells in another compartment against a destructive immune response. Alternatively, one compartment of a device can be loaded with cells that produce and secrete a molecule that improves the survival and function of cells in another compartment.

The compartments of a device can be combined prior to or at the time of transplantation. The cell-loaded compartment can be maintained under suitable culture conditions before joining with another compartment to allow proliferation and differentiation of the cells, or extracellular matrix production from the cells.

The present invention is also directed towards methods of treating disease, particularly diabetes, in a mammal utilizing a delivery device of the invention.

### BRIEF DESCRIPTION OF THE DRAWINGS

**Figure 1** represents a schematic of a three-compartment device where each compartment fits tightly inside the external adjoining compartment.
**Figure 2** is a picture of a two-compartment device where the inner compartment is comprised of fibrous nonwoven Vicryl® reinforced with the polymer PGA/PCL (65/35) and the outer compartment is comprised of fibrous nonwoven Vicryl®.
**Figure 3** is a representation of a two-compartment device where the inner compartment is comprised of a 5% PGA/PCL foam loaded with differentiation factors and the outer compartment is comprised of fibrous nonwoven Vicryl® loaded with undifferentiated or partially differentiated cells.
**Figure 4** is a representation of a two-compartment device where the inner compartment is loaded with the angiogenic factor VEGF-121 and the outer compartment is loaded with islets of Langerhans. The controlled release of VEGF-121 from the inner compartment creates a chemical gradient attracting endothelial cells into the device to initiate vasculature.
**Figure 5** is a representation of a two-compartment device where the inner compartment is loaded with GLP-1 or Exendin-4 and the outer compartment is loaded with insulin producing cells.
**Figure 6** is a picture of a two-compartment device where the inner compartment is loaded with VEGF-121. The device is surrounded with a layer of collagen gel containing rat aorta endothelial cells.
**Figure 7** is a microscopic image at 40x of the complex comprised of a two-compartment device loaded with a blank vehicle and surrounded by a layer of collagen gel loaded with endothelial cells. The complex is stained with a fluorescent nuclear stain to localize cells throughout the gel and associated compartments. Without VEGF-121, the cells organize in a ring-like pattern in the collagen gel.
**Figures 8-9** are microscopic images at 40x of the complex comprised of a two-compartment device loaded with VEGF-121 and surrounded by a layer of collagen gel loaded with endothelial cells. The complex is stained with a fluorescent nuclear stain to localize cells throughout the gel and associated compartments. In the presence of VEGF-121, the endothelial cells abandon a ring-like geometry and migrate towards the outer compartment (indicated by arrows).
**Figure 10** is a calibration curve showing the change in TNF-α secretion from LPS-stimulated PBMC relative to a change in soluble p38 inhibitor (RWJ 67657).
   **Figure 11** is a graph representing dose dependent inhibition of TNF-α secretion from LPS-stimulated PBMC in the presence of compartments loaded with a p38 inhibitor (RWJ 67657). The PBMC were loaded onto the inner compartment and the inhibitor compound was loaded onto the outer compartment.

### DETAILED DESCRIPTION OF THE INVENTION

The present invention provides biocompatible delivery devices that are uniquely designed to utilize two or more separately prepared compartments to host multiple biological entities. A principal feature of the devices of the present invention is the flexibility in the design, fabrication and loading of the compartments separately. A compartment can be designed and fabricated to address the specific need of a biological entity to be hosted by that compartment. Another desirable feature of the devices of the present invention is the ability to combine the compartments at the time of transplantation to permit interactions between the biological entities in separate compartments.

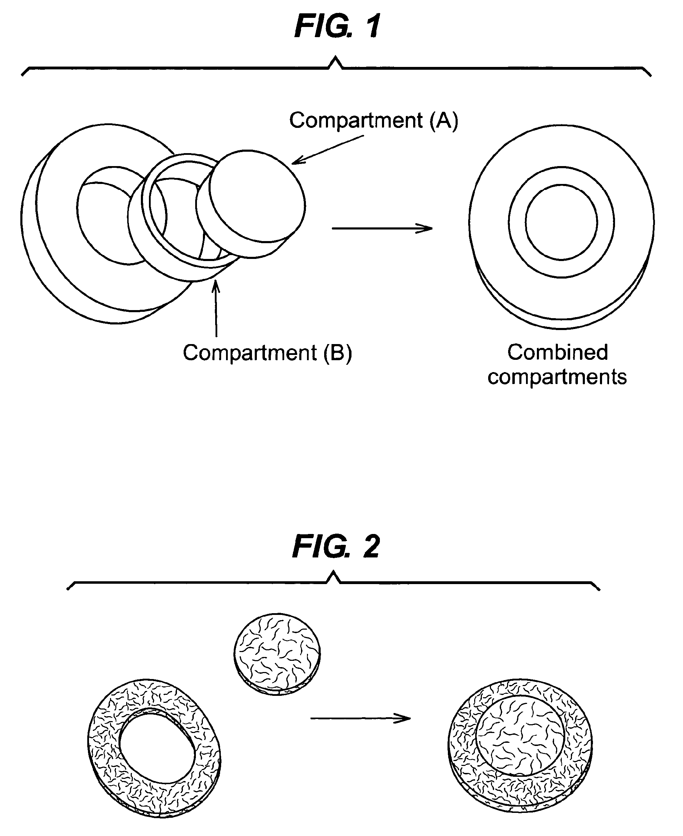

To illustrate the features of the present invention, an example of a device of the present invention is depicted in **Figure 1**, showing a three-compartment device where each compartment fits tightly inside the external adjoining compartment. Each compartment can be designed and fabricated to specifically host a particular biological entity. For example, compartment (A) in Figure 1 is fabricated to host cells, whereas compartment (B) is prepared to incorporate a therapeutic compound. Alternatively, compartment (A) is prepared to accommodate a therapeutic compound, whereas compartment (B) is prepared to accommodate cells.

The term "biological entity" is used herein as a general term and refers to any biologically active material suitable for use in implantation, including but not limited to compounds and cells, either in a single-cell suspension or a cell cluster.

One or more compartments of a device of the present invention can host cells ("cellular compartment"). A cellular compartment is designed to optimize cell survival and function in a transplanted graft. An important characteristic of a cellular compartment is its porosity. The term "porosity" refers to the ratio of the volume of all the pores within a compartment to the total volume of the compartment. Appropriate porosity allows diffusion of nutrients and oxygen, which is necessary for cell survival, especially in the initial stage after transplantation. Appropriate porosity and pore size also allow tissue infiltration to establish a permanent vasculature network. The porosity and pore size of a cellular compartment may vary depending on the type of cells being transplanted and the dimensions of its constituents. Generally speaking, a compartment suitable for hosting cells has an average pore diameter in the range of from about 50 to about 1,000 microns, preferably, from about 50 to about 500 microns; and has a porosity in the range of 70% to 95%, preferably about __________% (**Please fill in**).

A cellular component of a device of the present invention can host mammalian cells, including but not limited to, bone marrow cells, smooth muscle cells, stromal cells partially or fully differentiated glucose responsive insulin secreting cells, stem cells, mesenchymal stem cells, synovial derived stem cells, embryonic stem cells, blood vessel cells, chondrocytes, osteoblasts, precursor cells derived from adipose tissue, bone marrow derived progenitor cells, kidney cells, intestinal cells, islets, beta cells, Sertoli cells, peripheral blood progenitor cells, ductal cells, acinar cells, fibroblasts, glomus cells, keratinocytes, nucleus pulposus cells, annulus fibrosus cells, fibrochondrocytes, stem cells derived from placenta, amniotic epithelium, amniotic fluid, umbilical cord, chorion, villus, cord or cord blood, stem cells isolated from adult tissue, oval cells, neuronal stem cells, glial cells, macrophages and genetically transformed cells, or combinations of the above.

In one embodiment, the cells loaded into a cellular compartment produce a factor or a group of factors that are beneficial to cells of a different type loaded into another compartment of the same device so as to improve the survival, proliferation, differentiation, or function of such cells in such other compartment.

Cells are loaded into a cellular compartment using methods known to those skilled in the art. See, e.g., **Please provide a couple of references.** The cells can be maintained in the compartment for a short period of time (<1 day) prior to implantation, or cultured for a longer time period (>1 day) to allow for cell proliferation and extracellular matrix synthesis within the compartment prior to implantation.

In one embodiment, a cellular compartment is treated with factors that facilitate cell seeding and enhance cell attachment, for example, fibronectin, collagen, laminin and other extracellular matrices.

In another embodiment, a compartment loaded with cells is maintained *in vitro* using appropriate culturing techniques to allow the cells sufficient time to anchor, proliferate, or differentiate into functional cells prior to transplantation.

Although a cellular compartment is primarily designed and fabricated to host cells, such compartment can also include a bioactive compound so long as the inclusion of the compound does not interfere with the attachment, survival and function of the cells. However, it is desirable to incorporate the compound(s) into a separate compartment, which is independently designed to achieve optimum incorporation and release kinetics of the compound(s).

Accordingly, one or more compartments of a device of the present invention are designed and prepared as a compound compartment, primarily to achieve optimum incorporation and release kinetics of a compound(s). The characteristics of a compound compartment and the technique for loading a compound may vary depending on the physical nature of the compound, its mechanism of action, and desired release kinetics.

The term "bioactive compound" refers to small molecules, peptides, proteins, growth factors, differentiation factors, or combinations thereof. Bioactive compounds include any biological or synthetic factor that promotes attachment, proliferation, differentiation, and extracellular matrix synthesis of targeted cell types. It also includes, but is not limited to, anti-rejection agents, analgesics, antioxidants, anti- apoptotic agents such as erythropoietin, anti- inflammatory agents such as anti-tumor necrosis factor alpha, anti-CD44, anti- CD3, anti-CD154, p38 kinase inhibitor, JAK-STAT inhibitors, anti-CD28, acetoaminophen, cytostatic agents such as rapamycin, anti-IL2 agents, and combinations thereof.

In one embodiment, the compound is a small molecule that can be loaded during the fabrication process of the compound compartment. In another embodiment, the compound is a large biological factor that is sensitive to the fabrication process, and can be loaded at a later stage via adsorption, coating or a variety of other loading techniques known to those skilled in the art.

Examples of large biological factors that can be loaded into a compound compartment include growth factors, extracellular matrix proteins, and biologically relevant peptide fragments such as, but not limited to, members of the TGF-β family including TGF-β1, 2, and 3, bone morphogenic proteins (BMP-2, -4, 6, -12, and -13), fibroblast growth factors-1 and -2, platelet-derived growth factor-AA, and -BB, platelet rich plasma, insulin growth factor (IGF-I, II), growth differentiation factor (GDF-5, -6, -8, -10) vascular endothelial cell-derived growth factor (VEGF), exendin 4, (monocyte chemoattractant protein-1) (MCP1), pleiotrophin, endothelin, nicotinamide, glucagon like peptide-I and II, parathyroid hormone, tenascin-C, tropoelastin, thrombin- derived peptides, laminin, biological peptides containing cell- and heparin-binding domains of adhesive extracellular matrix proteins such as fibronectin and vitronectin, and combinations thereof.

The compartments of the present devices are made of biocompatible materials. By "biocompatible" is meant that the device of the present invention does not substantially adversely affect any desired characteristics of the biological entity to be seeded or incorporated within the device, or the cells or tissues in the area of a living subject where the device is to be implanted, or any other areas of the living subject.

By "a living subject" is meant to include any mammalian subject, including a primate, porcine, canine or murine subject, and particularly a human subject.

A compartment can be fully or partially biodegradable, and one or all the compartments of a device can be made biodegradable or non-biodegradable depending on the application. By "biodegradable" or "bioabsorbable" is meant that after the device is delivered inside the body of a living subject, the device will be gradually degraded or absorbed by the body, or passed from the body.

Those skilled in the art will appreciate that the selection of a suitable material for forming a particular compartment of the devices of the present invention depends on a number of factors. The more relevant factors in the selection of the appropriate material include bioabsorption (or biodegradation) kinetics; *in vivo* mechanical performance; cell response to the material in terms of cell attachment, proliferation, migration, differentiation, and biocompatibility. Other relevant factors, which to some extent dictate the *in vitro* and *in vivo* behavior of the material, include the chemical composition, the spatial distribution of the constituents, the molecular weight, the degree of crystallinity, and the monomer content (**what do you mean by this term?)** in the case of polymeric materials.

Suitable materials for making the compartments of the present devices include biocompatible metals such as stainless steel, cobalt chrome, titanium and titanium alloys; or of bio-inert ceramics such as alumina, zirconia and calcium sulfate; biodegradable glasses or ceramics containing calcium phosphates.

Other materials suitable for making the compartments include non-biodegradable synthetic polymers, including but not limited to polyethylene, polymethylmethacrylte (PMMA), silicone, polyethylene oxide (PEO), polyethylene glycol (PEG), and polyurethanes.

The compartments of the present devices can also be made of biogradable synthetic polymers such as, without limitation, aliphatic polyesters, polyalkylene oxalates, polyamides, polycarbonates, polyorthoesters, polyoxaesters, polyamidoesters, polyanhydrides and polyphosphazenes. Aliphatic polyesters can be homopolymers or copolymers (random, block, segmented, tapered blocks, graft, triblock, etc.) having a linear, branched or star structure. Suitable monomers for making aliphatic homopolymers and copolymers may be selected from the group consisting of, but are not limited to, lactic acid, lactide (including L-, D-, meso and L,D mixtures), glycolic acid, glycolide, ε -caprolactone, p-dioxanone, trimethylene carbonate, δ-valerolactone, β- butyrolactone, ε-decalactone, 2,5-diketomorpholine, pivalolactone, α,α-diethylpropiolactone, ethylene carbonate, ethylene oxalate, 3-methyl-1,4-dioxane-2,5-dione, 3,3-diethyl-1,4-dioxan- 2,5-dione, γ-butyrolactone, 1,4-dioxepan-2-one, 1,5-dioxepan-2-one, 6,6-dimethyl- dioxepan-2-one and 6,8-dioxabicycloctane-7-one.

Preferred polymers include polylactic acid (PLA), polyglycolic acid (PGA), polycaprolactone (PCL), polydioxanone (PDO), trimethylene carbonate (TMC), polyvinyl alcohol (PVA), copolymers, or blends thereof.

Biodegradable, biocompatible elastomers are also particularly useful materials for making the devices of the present invention. Suitable elastomeric polymers include, but are not limited to, elastomeric copolymers of ε- caprolactone and glycolide with a mole ratio of ε-caprolactone to glycolide of from about 35/65 to about 65/35, more preferably from 35/65 to 45/55; elastomeric copolymers of ε-caprolactone and lactide where the mole ratio of ε -caprolactone to lactide is from about 35/65 to about 65/35 and more preferably from 35/65 to 45/55; elastomeric copolymers of lactide and glycolide where the mole ratio of lactide to glycolide is from about 95/5 to about 85/15; elastomeric copolymers of p- dioxanone and lactide where the mole ratio of p-dioxanone to lactide is from about 40/60 to about 60/40; elastomeric copolymers of ε- caprolactone and p-dioxanone where the mole ratio of ε-caprolactone to p-dioxanone is from about from 30/70 to about 70/30; elastomeric copolymers of p-dioxanone and trimethylene carbonate where the mole ratio of p-dioxanone to trimethylene carbonate is from about 30/70 to about 70/30; elastomeric copolymers of trimethylene carbonate and glycolide where the mole ratio of trimethylene carbonate to glycolide is from about 30/70 to about 70/30; elastomeric copolymers of trimethylene carbonate and lactide where the mole ratio of trimethylene carbonate to lactide is from about 30/70 to about 70/30, or blends thereof.

The compartments of the present devices can also be made of biodegradable biopolymers that are naturally occurring biological materials or derivatives thereof. Such biopolymers include, e.g., small intestine submucosa (SIS), hyaluronic acid, collagen, alginates, chondroitin sulfate, chitosan, and blends thereof.

In one embodiment, one or more compartments of a device are made of a fibrous scaffold, which is prepared from biocompatible metals, biodegradable glasses or ceramics, non-biodegradable or biodegradable polymers, as described herein above, or combinations thereof. The fibrous scaffold can be prepared by weaving, knitting, warped knitting (i.e., lace-like), dry laying, wet laying or braiding, and can be organized in a form selected from threads, yarns, nets, laces, felts or nonwoven mats.

In another embodiment, one or more compartments of a device is made of a porous, polymeric matrix prepared by conventional polymer processing techniques such as extrusion, cast molding, injection molding, and blow molding. In a specific embodiment, the porous matrix is in the form of a polymeric foam, which can be fabricated by a variety of techniques such as, for example, lyophilization, supercritical solvent foaming, gas injection extrusion, gas injection molding or casting with an extractable material (e.g., salts, sugar or similar suitable materials).

In still another embodiment, one or more compartments of a device are fabricated in the form of a composite of a polymeric matrix and a fibrous scaffold where the percentage of the polymeric matrix determines the overall porosity of the composite.

In accordance with the present invention, the materials and fabrication techniques are selected and tailored to produce a compartment that accommodates the specific biological entity or entities to be delivered by such compartment.

For example, a cellular compartment should be made such that the pore size and porosity of the compartment are optimal for uniform distribution of the cells in the compartment, for diffusion of nutrients and oxygen, and for ingrowth of cells to establish stable vasculature. Porosity and pore size can be controlled by a variety of means, including manipulating the density of the fibers in the fibrous component, the concentration or amount of the polymer solution used in forming the compartment. Additionally, a compartment, e.g., a fibrous scaffold, can be treated, after the fabrication of the scaffold, with factors that facilitate cell seeding and enhance cell attachment, for example, fibronectin, collagen, laminin and other extracellular matrices.

A compound compartment, on the other hand, should be made to have desired release kinetics of the compound. A variety of techniques have been developed to incorporate a compound into a polymeric porous device. The compound can be impregnated within the entire device via an injection technique disclosed in U.S. Patent No. 5,770,417. A device can also be submerged in a solution containing the compound such that the compound fills the interstices within the device. Alternatively, a scaffold device can be immersed in a solution containing the compound, and the solvent allowed to evaporate, thereby precipitating the compound on the surface of the scaffold, as disclosed in U.S. Patents 5,980,551 and 5,876,452. Additionally, a compound can be adhered to a scaffold by surface modification of the scaffold to allow better attachment, as achieved using techniques such as plasma irradiation (Kwok et al., *J*. *Controlled Release* 62: 301-311,1999). Another common technique is freeze drying, wherein the compound or a solution containing the compound is added to a polymer solution, and the solvent is sublimed leaving behind a polymer scaffold with the compound dispersed within. Organic solvents, such as an alcohol or ether with a relatively high melting point, can be used to facilitate the infiltration of soluble compounds into the porous matrix of a scaffold.

Although cellular compartments and compound compartments are typically designed and fabricated separately, certain compartments (e.g., a composite device) may be suitable for loading both cells and one or more bioactive compounds.

In a specific embodiment, the present invention provides a device composed of at least two compartments. The two compartments can be made in any geometrical shape and can be adjoined snugly with each other such that the biological entity in one compartment provides beneficial effect to the biological entity in the other compartment.

In a specific embodiment, one compartment of the device is a cellular compartment and another compartment is a compound compartment, and the two compartments can be adjoined snugly with each other to permit the interactions between the compound released from the compound compartment and the cells in the cellular compartment.

In a preferred embodiment, the device is comprised of an inner compartment that having a disk-like geometry and an outer compartment that has a ring-like geometry. A particularly preferred device is shown in **Figure 2,** where the inner compartment is made of a fibrous nonwoven Vicryl® reinforced with the polymer PGA/PCL (65/35) and the outer compartment is made of fibrous nonwoven Vicryl®.

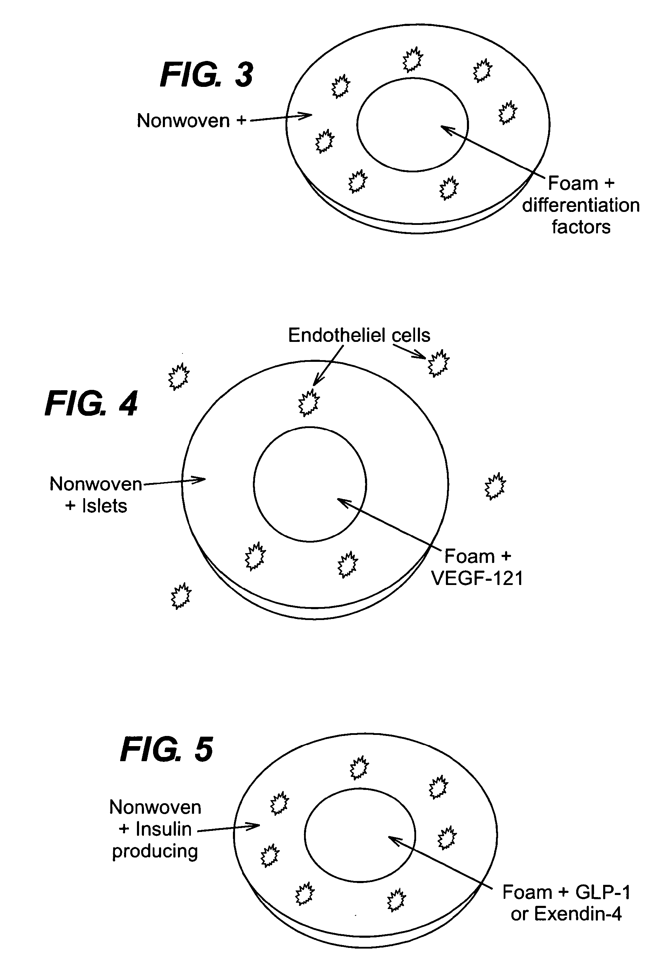

In a specific embodiment (**Figure 3**), the inner compartment is loaded with undifferentiated or partially differentiated cells, for example, insulin producing glucose responsive cells or precursors thereof. The outer compartment is loaded with factors that would guide the differentiation of the cells after transplantation into functional insulin producing cells.

In another specific embodiment (**Figure 4**), the inner compartment is loaded with an angiogenic factor, and the outer compartment is loaded with islets of Langerhans. The two compartments are then combined and transplanted. The release of the angiogenic factor creates a chemical gradient, which attracts endothelial cells and other cells involved in vascularization into the outer compartment to establish an intimate network of blood vessels surrounding the islets.

In yet another specific embodiment (**Figure 5**), a single entity or a combination of multiple compounds, such as GLP-1 and exendin-4, are loaded into an inner compartment to support survival and proliferation of insulin-producing cells loaded on an outer compartment.

In still another embodiment, the device includes at least two cellular compartments prepared separately for loading two different types of cells, where the two compartments can be joined snugly with each other such that the cells in one compartment provides beneficial effect to the cells in the other compartment.

In a specific embodiment, the device is comprised of an inner compartment that having a disk-like geometry and an outer compartment that has a ring-like geometry, where the inner compartment is loaded with islets of Langerhans and the outer compartment is loaded with Sertoli cells. The Sertoli cells can improve the survival of the islets and prevent or reduce an immune response upon implantation.

In another specific embodiment, one compartment of a device is loaded with cells that produce and secrete a molecule that improves the survival and function of cells in another compartment. **Please provide a specific example here**.

The compartments of a device can be combined prior to implantation and maintained under suitable conditions for a period of time prior to implantation. Alternatively, the compartments are combined and are implanted shortly thereafter. Additionally, one or more compartments can be implanted first, and the remaining compartment(s) can be implanted at a later time.

In a further aspect of the present invention, a device as described hereinabove is utilized for the treatment of diabetes mellitus.

Those skilled in the art will realize that a key feature of the current invention lies the ability to assemble a device with compartments prepared to address the specific requirements of the biological entities to be delivered. The devices of the present invention allow for the incorporation of a variety of therapeutic compounds, each with distinct release profiles, without compromising the porosity necessary for the survival and function of transplanted cells.

The following examples are illustrative of the principles and practice of the invention, although not limiting the scope of the invention. Numerous additional embodiments within the scope and spirit of the invention will become apparent to those skilled in the art.

### EXAMPLES

In the examples, the polymers and monomers were characterized for chemical composition and purity (NMR, FTIR), thermal analysis (DSC) and molecular weight by conventional analytical techniques.

Inherent viscosities (I.V., dL/g) of the polymers and copolymers were measured using a 50 bore Cannon-Ubbelhode dilution viscometer immersed in a thermostatically controlled water bath at 30°C, utilizing chloroform or hexafluoroisopropanol (HFIP) as the solvent at a concentration of 0.1 g/dL.

Certain abbreviations are used. These include PCL to indicate polymerized ε-caprolactone; PGA to indicate polymerized glycolide and PLA to indicate polymerized (L) lactide. Additionally, the ratios in front of the copolymer identification indicate the respective mole percentages of each constituent.

### Example 1: Fabrication of a polymeric foam outer compartment

The polymer used to manufacture the foam compartment was a 35/65 PCUPGA copolymer produced by Birmingham Polymers Inc. (Birmingham, AL), with an I.V. of 1.45 dUg. A 5/95 weight ratio of 35/65 PCUPGA in 1,4-dioxane solvent was weighed out. The polymer and solvent were placed into a flask, which in turn was put into a water bath and stirred for 5 hours at 70oC to form a solution. The solution was then filtered using an extraction thimble (extra coarse porosity, type ASTM 170-220 (EC)) and stored in a flask at room temperature.

A laboratory scale lyophilizer, or freeze dryer, (Model Duradry, FTS Kinetics, Stone Ridge, NY), was used to form the compartment. The polymer solution was added into a 4-inch by 4-inch aluminum mold to a height of 2 mm. The mold assembly was then placed on the shelf of the lyophilizer and the freeze dry sequence begun. The freeze drying sequence used in this example was: 1) -17°C for 60 minutes, 2) -5°C for 60 minutes under vacuum 100 mT, 3) 5°C for 60 minutes under vacuum 20 mT, and 4) 20°C for 60 minutes under vacuum 20 mT.

After the cycle was completed, the mold assembly was taken out of the freeze dryer and allowed to degas in a vacuum hood for 2 to 3 hours. A foam sheet was then removed from the mold and an 8mm dermal biopsy punch (Miltex Inc. New York, NY) was used to cut a disk from the foam sheet. Another dermal biopsy punch that is 5mm in diameter was used to cut a centric 5mm disk in the previously cut 8mm disk leaving behind a ring with an inner diameter of 5mm and an outer diameter of 8mm.

### Example 2: Fabrication of a fibrous inner compartment

A needle-punched nonwoven mat (2 mm in thickness) composed of a 90/10 PGA/PLA (vicryl® Ethicon Inc.) fiber was made as described below. A copolymer of PGA/PLA (90/10) was melt-extruded into continuous multifilament yarn by conventional methods of making yarn and subsequently oriented in order to increase strength, elongation and energy required to rupture. The yarns comprised filaments of approximately 20 microns in diameter. These yarns were then cut and crimped into uniform 2-inch lengths to form 2-inch staple fibers.

A dry lay needle-punched nonwoven mat was then prepared utilizing the 90/10 PGA/PLA copolymer staple fibers. The staple fibers were opened and carded on standard nonwoven machinery. The resulting mat was in the form of webbed staple fibers. The webbed staple fibers were needle punched to form the dry lay needle-punched, fibrous nonwoven mat.

The mat was scoured with iso-propanol for 60 minutes, followed by drying under vacuum. A 5mm biopsy punch was used to cut a disk from the fibrous mat.

### Example 3: Fabrication of a foam / fibrous composite inner compartment

The polymer used to manufacture the foam component was a 35/65 PCUPGA copolymer produced by Birmingham Polymers Inc. (Birmingham, AL), with an I.V. of 1.45 dL/g. A 0.5/99.5 weight ratio of 35/65 PCUPGA in 1,4-dioxane solvent was weighed out. The polymer and solvent were placed into a flask, which in turn was put into a water bath and stirred for 5 hours at 70°C to form a solution. The solution then was filtered using an extraction thimble (extra coarse porosity, type ASTM 170-220 (EC)) and stored in a flask.

A laboratory scale lyophilizer, or freeze dryer, (Model Duradry, FTS Kinetics, Stone Ridge, NY), was used to form the composite sheet. Approximately 10 ml of the polymer solution was added into a 4-inch by 4-inch aluminum mold to cover uniformly the mold surface. The needle-punched fibrous mat prepared in Example 2 was immersed into the beaker containing the rest of the solution until fully soaked and was then placed in the aluminum mold. The remaining polymer solution was poured into the mold so that the solution covered the nonwoven mat and reached a height of 2 mm in the mold. The mold assembly then was placed on the shelf of the lyophilizer and the freeze-drying sequence begun. The freeze drying sequence used in this example was: 1) -17°C for 60 minutes, 2) -5°C for 60 minutes under vacuum 100 mT, 3) 5°C for 60 minutes under vacuum 20 mT, and 4) 20°C for 60 minutes under vacuum 20 mT.

After the cycle was completed, the mold assembly was taken out of the freeze drier and allowed to degas in a vacuum hood for 2 to 3 hours. The composite sheet was then removed from the mold and a dermal biopsy punch was used to cut a 5mm disk from the sheet.

### Example 4: Incorporation of VEGF-121 into a composite inner compartment and its chemoattractive effect on endothelial cells.

Several composite inner compartments were fabricated as indicated above in Example 3. The compartments were then sterilized using EtO method of sterilization. A 0.5mg/ml solution of VEGF-121 in a co-solvent system of PBS and tertiary butanol was prepared with the tertiary butanol to PBS ratio being 6%. An aliquot of 20µl of this solution was pipetted onto each of three composite inner compartments. Within 10 seconds, VEGF solutions completely infiltrated the scaffolds, which were then frozen and the solvents sublimed. Similar procedure was used to load 3 other inner compartments with blank vehicles as controls.

Contracting collagen gels were made using type I rat tail collagen (3.69 mg/ml, BD Biosciences) and primary rat thoracic aorta endothelial cells (0.5X106/mL). Medium (Endothelial Growth Medium-2, Cambrex), collagen, neutralizing medium (medium containing 0.1 N NaOH), and cells were mixed in a 4:2:1:1 ratio respectively. Six ml of the collagen/cell mixture was pipetted into each well of a 6-well low cluster plate (Costar) and allowed to solidify at 37°C, 5% CO₂ for 2-4 hours prior to the addition of 3ml medium. After two days, the gels had contracted by approximately 4mm.

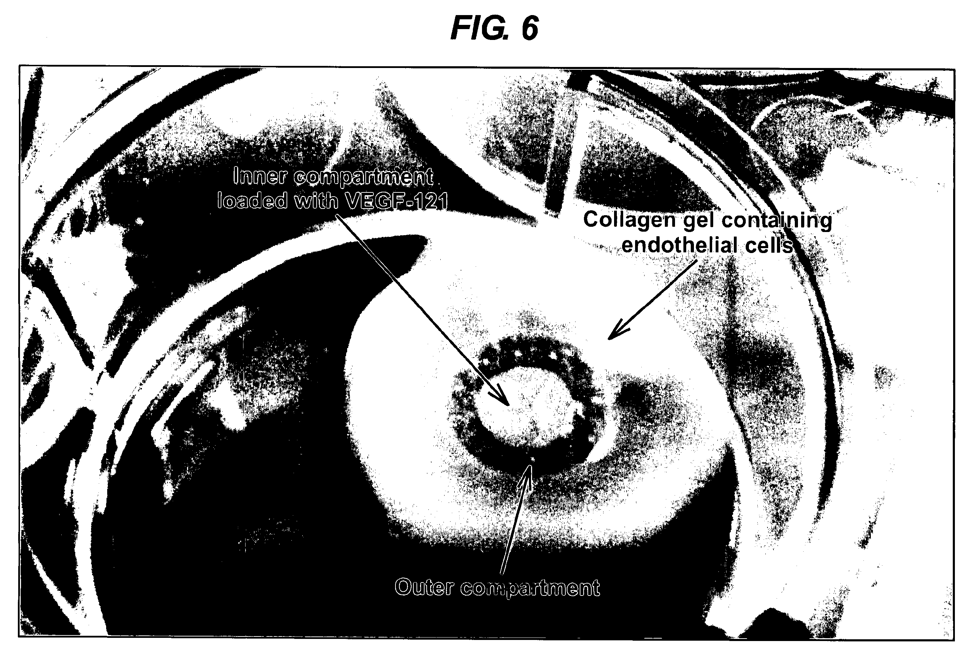

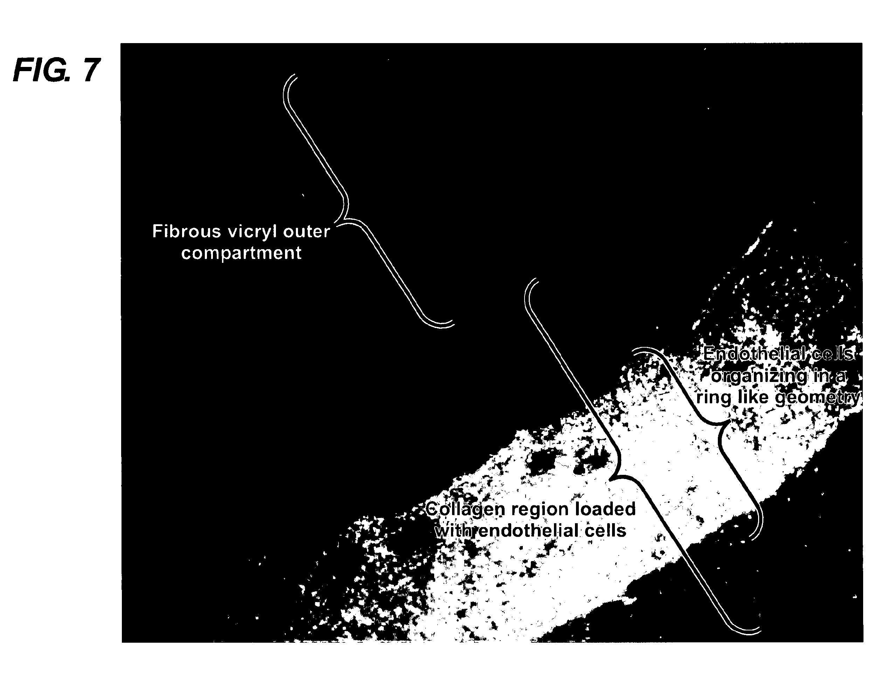

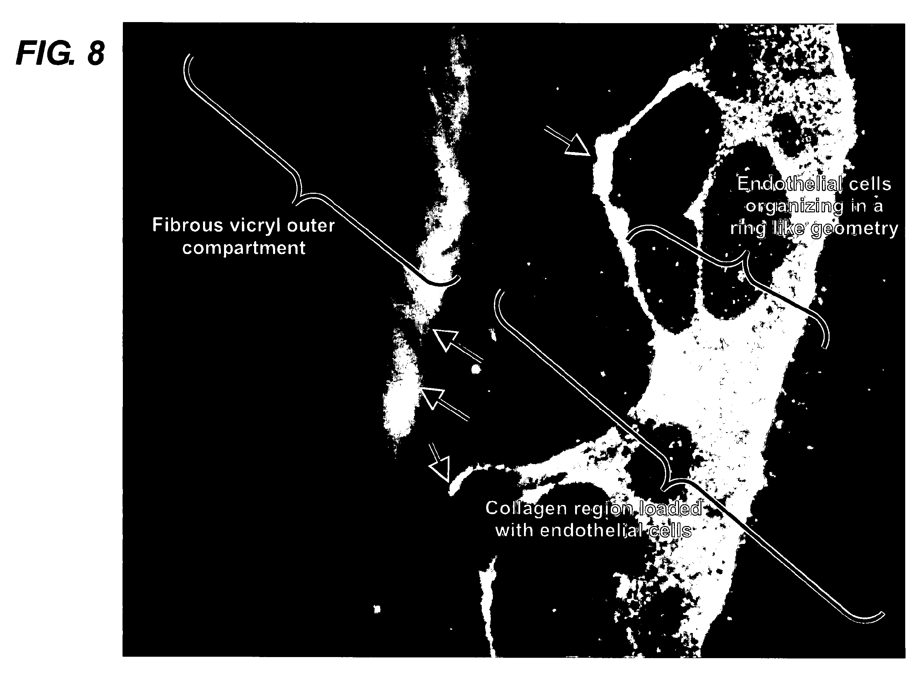

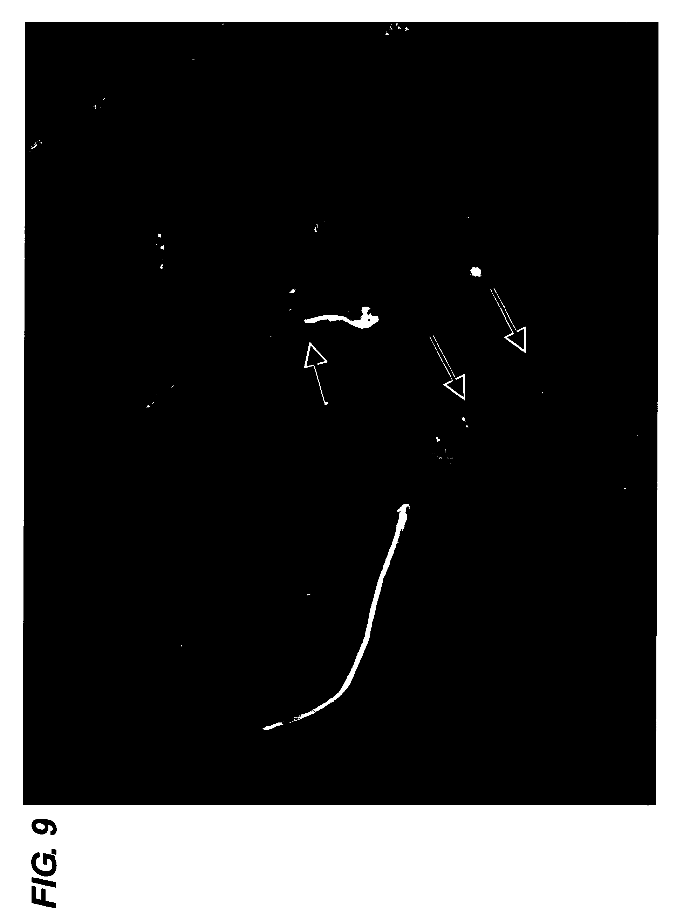

An 8 mm biopsy punch was used to remove the center of the gel from all samples. Each inner compartment (d=5mm)(loaded either with VEGF-121 or a blank vehicle) was combined with an outer fibrous compartment (d=8mm), fabricated essentially as described in Example 2. Finally each two-compartment device was inserted into the space prepared in the collagen gel (**Figure 6**). The original piece of excised collagen was placed over the device and the composite was weighed down with a well insert. Medium with VEGF removed was used to feed the gels. Medium was changed approximately every three days and later analyzed for VEGF content. At day 15, some samples were fixed in formalin for histological analysis while others were stained with 4', 6-diamidino-2-phenylindole, dihydrochloride (DAPI, Molecular Probes), a fluorescent nuclear stain to visualize cells throughout the gel and scaffolds. **Figure 7** shows that endothelial cells in the collagen gel arranged themselves in a circle-like geometry surrounding the device. In devices that incorporated VEGF-121 (**Figures 8-9**), the cells have abandoned their uniform circular geometry with noticeable migration towards the outer compartment. This observation leads to the conclusion that VEGF-121, which was released from the inner compartment, created a chemical gradient to attract cells towards the outer compartment.

### Example 5: Assessing the Functional Activity of a p38 Inhibitor in an outer compartment

Outer compartments were made from a composite matrix similar to the one prepared in Example 3. The compartments were loaded with three different amounts (10.99 ng, 109.9 ng, or 1099 ng) of p38 kinase inhibitor (JNJ 3026582, also known as RWJ 67657). The chemical structure of this compound (4-[4-(4-fluorophenyl)-1-(3-phenylpropyl)-5-(4-pyridinyl)-1H-imidazol-2-yl]-3-butyn-1-ol) has been previously documented (Wadsworth et al., J. Pharm. Exp. Ther. 291:680-687, 1999).

The inner compartments were prepared from a non-woven Vicryl® fibrous matrix fabricated as described in Example 2. Mononuclear cells were freshly isolated from human peripheral blood, counted and adjusted to a final cell number in RPMI-1640 (Invitrogen Life Technologies, Carlsbad, CA) with 1% fetal bovine serum (FBS, HyClone, Logan, UT). A total of 1x10⁶ cells in a final volume of 50ul were seeded onto the inner compartments.

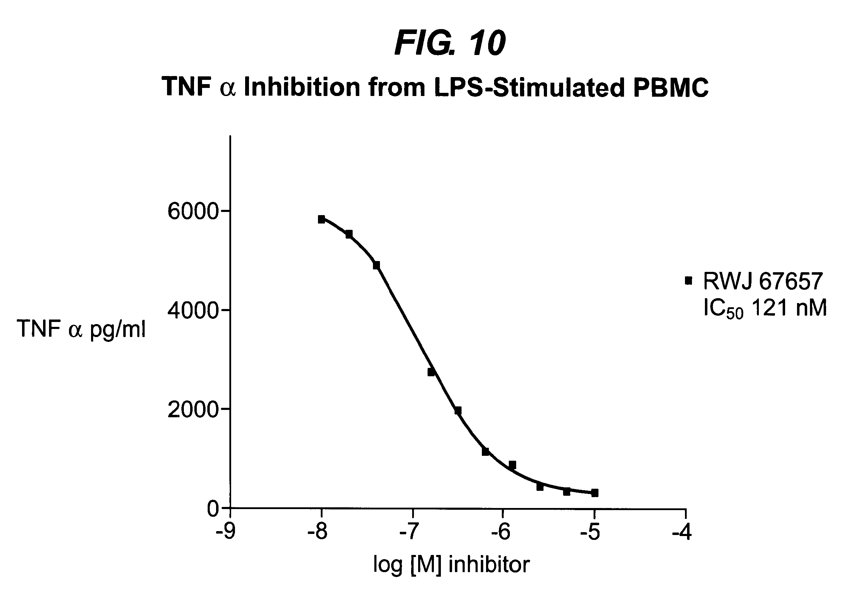

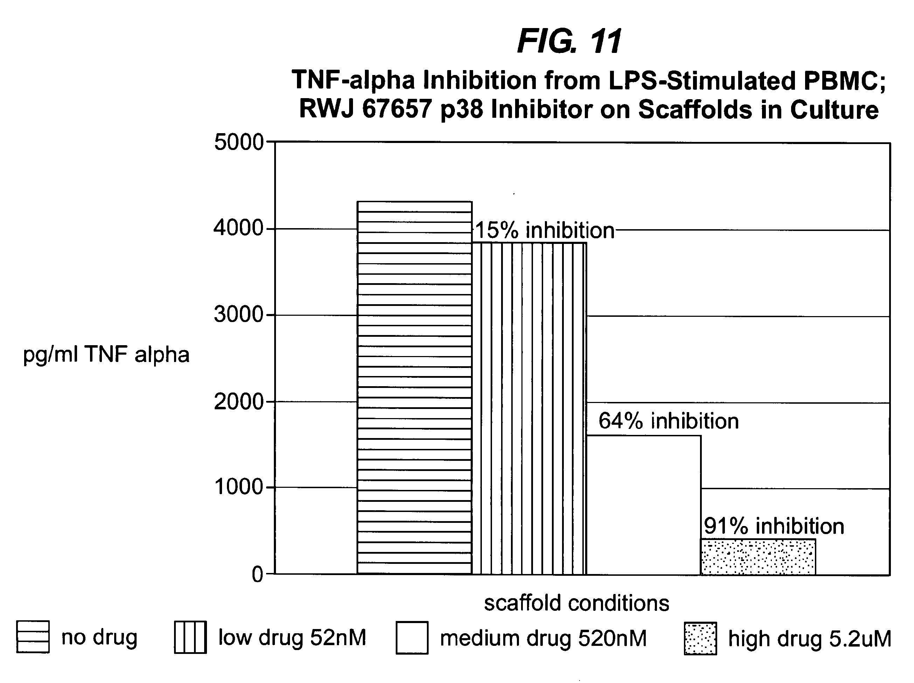

The two compartments were combined and pre-cultured for 1 hour in a 24-well plate with 0.5 ml culture medium. A stimulus of LPS (**FULL NAME?)** (10ng/ml; Sigma, St. Louis, MO) was added to each well and incubated overnight at 37°C, 5%CO₂. Supernatant from each well was collected and analyzed for TNF-α content using a standard commercial ELISA kit (R&D Systems, Minneapolis, MN). Control wells for assay were seeded with an identical number of cells and a titrated amount of drug in solution without the presence of the device (**Figure 10**). TNF-α secretion was inhibited in a dose-dependent manner from compartments impregnated with a p38 inhibitor within an inhibition range similar to equivalent amounts of soluble drug (**Figure 11**).

## Claims

1. A biocompatible, implantable, partially or fully biodegradable delivery device comprising at least two compartments, wherein said two compartments are prepared separately for delivering at least two distinct biological entities.

2. The device of claim 1, wherein said two compartments can be physically combined with each other in a manner that permits a biological entity to be loaded in one compartment to benefit from a biological entity to be loaded in the other compartment.

3. The device of claim 2, wherein one of said two compartments is a cellular compartment, and the other one is a compound compartment, and wherein said two compartments can be combined in such a manner to permit a compound to be loaded in said compound compartment to benefit proliferation, differentiation, survival or function of cells to be loaded in said cellular compartment.

4. The device of claim 2, wherein said two compartments are both cellular compartments, and wherein said two compartments can be combined in such a manner to permit cells to be loaded in one compartment to benefit proliferation, differentiation, survival or function of cells to be loaded in the other compartment.

5. The device of claim 3, wherein said compound compartment has been loaded with a compound.

6. The device of claim 5, wherein said compound promotes attachment, proliferation or differentiation of cells loaded in an adjoining cellular compartment; or promotes extracellular matrix synthesis.

7. The device of claim 5, wherein said compound is selected from anti-rejection agents, analgesics, antioxidants, anti-apoptotic agents, or antiinflammatory agents.

8. The device of claim 5, wherein said compound is selected from the group consisting of members of the TGF-β family, bone morphogenic proteins, fibroblast growth factors-1 and -2, platelet-derived growth factor-AA and -BB, platelet rich plasma, insulin growth factors), growth differentiation factors, vascular endothelial cell-derived growth factor (VEGF), exendin 4, monocyte chemoattractant protein-1 (MCP1), pleiotrophin, endothelin, nicotinamide, glucagon like peptide-I and II, parathyroid hormone, tenascin-C, tropoelastin, thrombin- derived peptides, laminin, biological peptides comprising cell- and heparin-binding domains of adhesive extracellular matrix proteins, and combinations thereof.

9. The device of claim 3 or claim 4, wherein said cellular compartment or compartments have been loaded with cells.

10. The device of claim 9, wherein said cells are selected from the group consisting of partially or fully differentiated glucose responsive insulin secreting cells, bone marrow cells, smooth muscle cells, stromal cells, stem cells, mesenchymal stem cells, synovial derived stem cells, embryonic stem cells, blood vessel cells, chondrocytes, osteoblasts, precursor cells derived from adipose tissue, bone marrow derived progenitor cells, kidney cells, intestinal cells, islets, beta cells, Sertoli cells, peripheral blood progenitor cells, fibroblasts, glomus cells, keratinocytes, nucleus pulposus cells, annulus fibrosus cells, fibrochondrocytes, stem cells derived from placenta, amniotic epithelium, amniotic fluid, umbilical cord, cord or cord blood, stem cells isolated from adult tissue, oval cells, neuronal stem cells, glial cells, macrophages, and combinations of the above.

11. The device of claim 2, wherein said two compartments are combined at the time of implantation.

12. The device of claim 3 or claim 4, wherein the cellular compartment or compartments are seeded with cells and are maintained *in vitro* for a period of time under appropriate culture conditions prior to implantation.

13. A method of treating a disease in a mammal, comprising implanting a biocompatible, partially or fully biodegradable delivery device which comprises at least two compartments, wherein said two compartments are prepared separately and are loaded separately with distinct biological entities that contribute to the treatment.

14. The method of claim 13, wherein said disease is insulin dependent diabetes.
